# FASCICULE DE BREVET EUROPEEN

(11) **EP 2 919 592 B1**
(45) Date de publication et mention de la délivrance du brevet: **21.02.2018**
(21) Numéro de dépôt: 13803137.2
(22) Date de dépôt: 13.11.2013
(51) Int. Cl.: A61K 31/718, A23L 33/21, A23L 29/219, A61P 1/00

(54) **UTILISATION DE MALTODEXTRINES BRANCHEES DANS LE BIEN-ETRE INTESTINAL DU NOURRISSON**
VERWENDUNG VERZWEIGTER MALTODEXTRINE ZUR FÖRDERUNG DES INTESTINALEN WOHLBEFINDENS BEI SÄUGLINGEN
USE OF BRANCHED MALTODEXTRINS FOR THE INTESTINAL WELL-BEING OF INFANTS

(30) Priorité: 14.11.2012 FR 1260837
(43) Date de publication de la demande: 23.09.2015
(73) Titulaire: Roquette Frères, 62136 Lestrem (FR)
(72) Inventeur: GUERIN-DEREMAUX, Laëtitia, F-59850 Nieppe (FR); TEICHMAN-DUBOIS, Virginie, F-62840 Laventie (FR); WILS, Daniel, F-59190 Morbecque (FR)
(74) Mandataire: Cabinet Plasseraud
(86) Numéro de dépôt international: PCT/FR2013/052724
(87) Numéro de publication internationale: WO 2014/076415

(56) Documents cités:
- EP-A1- 1 006 128
- WO-A1-2012/089784
- FR-A1- 2 935 228
- FR-A1- 2 974 512
- US-A1- 2008 182 821
- US-A1- 2011 117 059
- US-A1- 2012 172 319

## Description

### DOMAINE DE L'INVENTION

La présente invention concerne l'utilisation de maltodextrines branchées pour améliorer la flore du colon du nourrisson et/ou de l'enfant en bas âge, et ainsi traiter ou prévenir les coliques, les entérocolites et donc améliorer et/ou favoriser de manière générale le bien-être intestinal des nourrissons et/ou des enfants en bas âge.

La présente invention concerne également une composition alimentaire destinée à l'alimentation du nourrisson et/ou de l'enfant en bas âge comprenant au moins un polysaccharide pour traiter ou prévenir les coliques, les entérocolites et améliorer et/ou favoriser le bien-être intestinal chez le nourrisson et/ou l'enfant en bas âge.

### ARRIERE-PLAN TECHNOLOGIQUE

La flore intestinale représente chez l'organisme hôte un réel écosystème, établissant des connexions étroites avec les épithéliums digestifs et le système immunitaire qui leur est associé. Les interactions entre les différentes bactéries tout comme entre les bactéries et leur hôte sont des facteurs de contrôle de l'équilibre de la flore microbienne et du développement de certains états pathologiques aigus ou chroniques. La flore digestive participe en outre à un certain nombre d'activités fonctionnelles et interfère avec la digestion et l'absorption de certains nutriments. Le rôle significatif de la flore intestinale en santé humaine a été reconnu au début du 20^{ème} siècle par la considération qu'un certain nombre de maladies pouvaient être la conséquence d'une rupture de l'équilibre de la flore intestinale, et que la restauration de cet équilibre était bénéfique en terme de santé. Cependant l'idée d'incorporer des micro-organismes dans l'alimentation humaine ou du moins d'utiliser les effets bénéfiques potentiels de la présence de ces micro-organismes semble remonter à la haute antiquité.

L'établissement de l'écosystème intestinal se fait pendant les premiers mois de la vie. En effet, les nourrissons sont dépourvus de flore intestinale à la naissance. *In utéro,* l'intestin est stérile.

Chez le nouveau-né, la colonisation du tube digestif est relativement stéréotypée durant les premiers jours, dépendant en partie de la composition de la flore vaginale et fécale maternelle ; elle est retardée chez les enfants nés par césarienne. Dès le troisième jour apparaissent des Bifidobactéries et des Lactobacilles, et dans une moindre mesure Bactéroïdes et Clostridiae. L'implantation de cette flore est directement dépendante du type d'alimentation reçue, des conditions environnementales et de la prescription éventuelle d'antibiotiques.

A la fin du premier mois, des différences très nettes existent dans la composition de la flore intestinale selon le type d'alimentation reçue par le nouveau-né. Ainsi, la flore intestinale des enfants allaités est presque exclusivement composée de Bifidobactéries. Les facteurs bifidogènes du lait maternel sont nombreux et ne sauraient être limités aux seuls galacto-oligo-saccharides.

En revanche, la flore des bébés nourris avec des laits infantiles est riche en Entérobactéries et en bactéries Gram-négatif. Dès que l'alimentation commence à être diversifiée, la différence entre la flore des enfants nourris au sein et celle des enfants nourris artificiellement s'estompe avec augmentation de la concentration en *Escherichia coli, Enterococcaceae* et *Clostridiaceae.* Entre l'âge de 1 an et 2 ans, l'enfant acquiert une flore intestinale quasiment identique à celle de l'adulte. Cette microflore demeure relativement stable au cours de la vie, sauf en cas de changements drastiques de l'alimentation, d'antibiothérapie, ou de modulation par l'ingestion de prébiotiques ou probiotiques.

Le terme colique du nourrisson (CN) fait usuellement référence à une entité clinique caractérisée par la survenue paroxystique chez un nourrisson de moins de trois mois de pleurs prolongés et de phases d'agitation dont la cause est présumée être d'origine intestinale. Les CN sont souvent dues au fait que, jusqu'alors nourri directement via le cordon ombilical et sa circulation sanguine, le nourrisson a très peu fait usage de son tube digestif et encore moins de son estomac. Les premiers mois, l'adaptation du système digestif, induite par la digestion, produit des douleurs digestives chez le bébé appelées coliques. L'ingestion d'air lors de l'allaitement aggrave le phénomène puisque cela produit des gaz. On parle de coliques uniquement si le nourrisson est par ailleurs en bonne santé et son poids normal. Elles surviennent généralement en fin d'après-midi, après un repas. L'enfant est agité, il replie les jambes contre son ventre, pleure et se montre inconsolable jusqu'à ce qu'il émette enfin un gaz. Les CN n'ont pas nécessairement d'incidence sur les selles du nourrisson, qui restent généralement normales

Le terme entérocolite, et plus précisément le terme entérocolite ulcéro-nécrosante (ECUN) désigne une pathologie plus grave, voire mortelle dans les cas les plus sévères. Elle résulte d'agressions diverses souvent intriquées. L'ECUN est une inflammation de l'intestin grêle ou du colon entraînant des selles fréquentes et peu consistantes, qui peuvent être douloureuses. Il s'agit d'une pathologie acquise du nouveau-né qui se définit par la survenue d'une nécrose multifocale de la paroi intestinale caractérisée par l'apparition de plage de nécrose ischémique et hémorragique pouvant entraîner des ulcérations susceptibles d'évoluer vers une perforation digestive. L'ECUN survient habituellement chez le nouveau-né prématuré, entre le 3e et le 10e jour de vie, elle est exceptionnelle chez le nouveau-né à terme. Elle résulte d'une agression multifactorielle de la muqueuse. La physiopathologie de l'ECUN est multifactorielle. La prématurité en est le principal facteur. Enfin, la stase digestive, l'alimentation entérale et l'immaturité immunologique du nouveau-né favoriseraient la survenue des ECUN. Des germes dangereux et pathogènes ont été isolés dans les hémocultures et le liquide péritonéal des nouveau-nés atteints d'ECUN : *Escherichia coli, Klebsiella pneumoniae, Pseudomonas aeruginosa, Staphylococcus aureus, Enterococcus faecalis* ainsi que des bactéries anaérobies responsables de formes sévères avec penumopatose. Une altération de l'état général est constante, mais le tableau clinique est variable allant de l'existence de signes digestifs isolés (abdomen ballonné et douloureux, vomissements bilieux, arrêt du transit) à celle de signes septiques (teint grisâtre, hypothermie, apnées, état de choc oligoanurique) dans les formes sévères.

Ainsi dans les premiers mois de la vie, la colonisation du tube digestif est une étape primordiale. D'ailleurs tout au long de la vie, un certain équilibre bactérien doit être assuré afin de maintenir un bien-être digestif constant chez l'enfant puis au stade adulte.

Le tube digestif est colonisé par une microflore très importante, implantée essentiellement au niveau colique, avec une grande diversité puisque composée de plus de 400 espèces différentes.

Le rôle d'une flore intestinale équilibrée est très important. La flore intervient dans la dégradation des polyosides, la fermentation des oses, la transformation de xénobiotiques, la protéolyse des acides aminés, la production d'hydrogène fermentaire et sa réutilisation, la production de gaz intestinaux, le métabolisme des acides biliaires, la production de mutagènes, mais aussi et surtout au niveau du développement du système immunitaire intestinal (gut associated lymphoïd tissue).

Ainsi l'équilibre de la flore intestinale participe à réguler le transit intestinal, à empêcher la pénétration d'antigènes hostiles et à éviter la pullulation de germes pathogènes. Les mécanismes évoqués pour rendre compte de l'inhibition de la flore pathogène sont multiples et souvent concomitants :
- production de substances inhibitrices tels que les acides organiques, notamment d'acide lactique, de peroxyde d'hydrogène (H2O2),
- occupation des sites d'adhésion sur la muqueuse intestinale ou sur le mucus la tapissant, contrecarrant ainsi la possibilité pour d'autres microorganismes de s'y fixer,
- dégradation de sites récepteurs de toxines,
- stimulation des fonctions immunitaires telles que la production d'immunoglobulines A (IgA). Les IgA sont un isotype d'anticorps majoritairement produit au niveau des muqueuses, où elles constituent une première ligne de défense immunitaire contre les toxines et les agents infectieux présents dans l'environnement. Dans l'intestin, la production d'IgA est fortement induite au cours de la colonisation des nouveau-nés par la flore intestinale, acquise au cours de l'accouchement et dans les heures qui suivent,
- compétition vis-à-vis des nutriments.

Un intérêt grandissant a vu le jour pour le contrôle écologique de la flore intestinale chez l'être humain, et plus particulièrement celle du nourrisson et de l'enfant en bas âge, par l'administration d'ingrédients pouvant être classés en trois types :
- les prébiotiques sont définis comme une substance non-digestible qui induit un effet physiologique bénéfique à l'hôte en stimulant de façon spécifique la croissance et/ou l'activité d'un nombre limité de populations bactériennes déjà établies dans le côlon. Cette définition ne met pas l'accent sur une population bactérienne en particulier. On admet de façon courante qu'un prébiotique augmente le nombre de bifidobactéries et de bactéries productrices d'acide lactique, car ces groupes de bactéries sont bénéfiques pour l'hôte. Ainsi les prébiotiques sont présents dans la lumière intestinale et stimulent la croissance sélective d'une flore considérée comme bénéfique en terme de santé. La clé de leur efficacité est qu'ils puissent être fermentés par une microflore intestinale spécifique dont le développement ainsi engendré sera bénéfique. Ce mode d'action implique donc une ingestion régulière de l'ingrédient concerné. De ce fait, un certain nombre d'études ont tenté d'identifier les facteurs potentiellement bifidogènes et au premier rang des candidats se sont inscrits certains oligosaccharides. Les oligosaccharides non digestibles résistent à l'hydrolyse des enzymes de la bordure en brosse et vont se comporter comme des substrats énergétiques pour certains éléments de la flore colique tels que les *lactobacilles* et les *bifidobactéries.* L'effet bifidogène des prébiotiques, ainsi que leur action immunitaire et métabolique potentielle font penser qu'ils puissent avoir un effet bénéfique notamment dans le cadre de la prévention des infections intestinales.
- Les probiotiques sont des micro-organismes, qui une fois ingérés sont susceptibles de demeurer vivants lors du transit intestinal et de modifier la flore intestinale en ayant un effet bénéfique démontré sur la santé, c'est là le facteur majeur qui en détermine l'efficacité. Les probiotiques utilisés sont des souches micro-organiques vivantes, productrices d'acide lactique tels que les lactobacilles, certains streptocoques et des bifidobactéries. Certaines souches sont d'autre part capables d'avoir un rôle immuno-régulateur, notamment en stimulant la production d'IgA et le pouvoir phagocytaire.
- Les symbiotiques sont définis comme un produit contenant à la fois un(des) probiotique(s) et un(des) prébiotiques(s). La présence de prébiotique(s) exerce un effet bénéfique sur la stabilité du (des) probiotique(s) dans le produit ainsi que sur sa survie et son implantation dans le tractus gastro-intestinal, tant que dure la présence du prébiotique.

Parmi les nombreux candidats prébiotiques, les plus connus et étudiés sont les fructanes (FOS : fructo-oligosacharides, oligofructose et inuline) et d'autres oligosides de galactose et transgalactose (GOS et TOS). Le lactose, qui échappe à la digestion dans l'intestin grêle, est également un prébiotique, et plusieurs travaux ont montré que du lactose pouvait parvenir dans le côlon chez le nourrisson. De nombreux autres glucides pourraient revendiquer l'appellation de prébiotiques (xylo-oligosaccharides, isomalto-oligosaccharides, gluco-oligosaccharides, etc.). Certains amidons résistants et des sucres alcools pourraient aussi avoir des propriétés prébiotiques. Le lactulose est également un prébiotique.

L'ingrédient prébiotique doit être parfaitement caractérisé. Les produits ou organismes à l'origine de l'ingrédient doivent être connus et caractérisés, qu'il s'agisse d'un ingrédient isolé d'un produit végétal, animal ou microbien, ou d'un ingrédient produit par synthèse chimique ou microbienne.

Le marché des produits alimentaires destinés aux nourrissons et aux enfants âgés de moins de trois ans, en bonne santé, a toujours été, depuis plus d'un siècle, sous l'impulsion constante des sociétés qui conçoivent et/ou commercialisent ces aliments, en constante évolution. Aujourd'hui, la réglementation européenne, transcrite en droit national, définit de façon plus élaborée la composition des deux types de préparations lactées autorisées à être commercialisées, soit les préparations pour nourrissons (pour les enfants nés à terme, de la naissance à 4 à 6 mois), et les préparations de suite (pour les nourrissons âgés de 4-6 mois à 12 mois). L'évolution de la composition de ces préparations lactées a ainsi constitué un progrès considérable sur le plan de la nutrition infantile. La meilleure adaptation aux besoins nutritionnels et aux capacités digestives du jeune enfant de la formule protéique, lipidique et glucidique, des teneurs en sels minéraux, oligoéléments et vitamines, a fait que lorsque la mère ne pouvait ou ne voulait pas allaiter, ou lorsque l'allaitement au sein après l'âge de 6 mois ne suffisait plus, ces préparations permettaient d'obtenir une croissance somatique normale, un développement psychomoteur satisfaisant, sans risque pathologique majeur.

Forte de ce constat et après de nombreux travaux de recherche, la société Demanderesse a eu le mérite de pallier toutes les exigences requises dans la nutrition infantile en proposant une nouvelle composition pour l'alimentation des nourrissons et/ou des enfants en bas âge. Plus précisément, la présente invention concerne le domaine des prébiotiques dans la nutrition infantile.

Des compositions de nutrition infantile pour traiter des pathologies digestives inflammatoires au moyen d'oligosaccharides sont proposées par US 2012/0172319. Par ailleurs, US 2008/0182821 propose l'utilisation de maltodextrines branchées pour obtenir un effet anti-inflammatoire et/ou analgésique au niveau du côlon.

### RESUME DE L'INVENTION

La présente invention est définie par les revendications et concerne l'utilisation d'une maltodextrine branchée définie plus loin pour améliorer la flore du colon du nouveau-né, et ainsi traiter ou prévenir les coliques, les entérocolites et éventuellement aussi améliorer et/ou favoriser de manière générale le bien-être intestinal du nourrisson. La présente invention concerne également une composition alimentaire destinée à l'alimentation du nourrisson comprenant au moins une maltodextrine branchée définie plus loin pour traiter ou prévenir les coliques, les entérocolites et éventuellement aussi améliorer et/ou favoriser le bien-être intestinal chez le nourrisson.

### DESCRIPTION DETAILLEE DES MODES DE REALISATION

La présente invention est définie par les revendications et concerne l'utilisation de maltodextrines branchées définies plus loin pour améliorer la flore du colon du nouveau-né, et ainsi traiter ou prévenir les coliques, les entérocolites et améliorer et/ou favoriser de manière générale le bien-être intestinal du nourrisson. Dans la présente invention, les termes ou expressions ci-dessous sont donnés avec leurs définitions telles que figurant dans la directive 2006/141/CE de la Commission Européenne du 22 décembre 2006, publiée au Journal Officiel de l'Union européenne le 30 décembre 2006, qui doivent par conséquent être prises en compte lors de la lecture et de l'interprétation de la présente description, des exemples et des revendications.

Ainsi, en adéquation avec la directive 2006/141/CE, article 2(a), le terme « nourrisson » désigne les enfants âgés de moins de douze mois. Le terme « enfant en bas âge » désigne selon l'article 2(b) les enfants âgés de un à trois ans. L'expression « préparations pour nourrissons » désigne selon l'article 2(c), les denrées alimentaires destinées à l'alimentation particulière des nourrissons pendant les premiers mois de leur vie et répondant à elles seules aux besoins nutritionnels de ces nourrissons jusqu'à l'introduction d'une alimentation complémentaire appropriée. Il faut comprendre ici que les nourrissons peuvent donc être nourris uniquement avec des préparations pour nourrissons, ou alors de façon mixte par lesdites préparations pour nourrissons utilisées en complément de l'alitement maternel. L'expression « préparation de suite » désigne selon l'article 2(d) les denrées alimentaires destinées à l'alimentation particulière des nourrissons lorsqu'une alimentation complémentaire appropriée est introduite et constituant le principal élément liquide d'une alimentation progressivement diversifiées de ces nourrissons.

L'invention concerne l'utilisation de maltodextrines branchées pour améliorer la flore du colon du nouveau-né. Au sens de la présente invention, l'amélioration de la flore du colon signifie une modulation de la microflore intestinale vers des populations bactériennes dites bénéfiques plus importantes au détriment des espèces bactériennes reconnues comme ayant un rôle dans les troubles digestifs décrits ci-dessus.

La présente invention concerne également une composition ou préparation alimentaire destinée à l'alimentation du nourrisson comprenant des maltodextrines branchées pour traiter ou prévenir les coliques, les entérocolites et améliorer et/ou favoriser le bien-être intestinal chez le nourrisson. Dans la présente demande, l'expression « composition pour la nutrition infantile » est employée et concerne aussi bien les compositions ou les préparations alimentaires destinées à l'alimentation des nourrissons que celles destinées aux enfants en bas âge, jusqu'à l'âge de trois ans environ.

Dans la présente invention, on entend par maltodextrines branchées (MDB), les maltodextrines spécifiques identiques à celles décrites dans le brevet EP 1.006.128-B1 dont la Demanderesse est titulaire. Ce brevet décrit l'obtention d'une maltodextrine branchée qui se distingue des maltodextrines standards notamment par leur richesse en liaisons glucosidiques 1-6. Ces MDB ont l'avantage de représenter une source de fibres indigestibles bénéfiques pour le métabolisme et pour l'équilibre intestinal.

Selon la présente invention, lesdites maltodextrines branchées sont caractérisées en ce qu'elles présentent:
- entre 15 et 50% de liaisons glucosidiques 1-6, préférentiellement entre 22% et 45%, plus préférentiellement entre 20% et 40%, et encore plus préférentiellement entre 25% et 35%,
- une teneur en sucres réducteurs inférieure à 20%, préférentiellement comprise entre 2% et 20%, plus préférentiellement entre 2,5% et 15%, et encore plus préférentiellement entre 3,5% et 10%,
- un indice de polymolécularité inférieur à 5, préférentiellement compris entre 1 et 4, plus préférentiellement entre 1,5 et 3, et
- une masse moléculaire moyenne en nombre Mn inférieure à 4500 g/mole, de préférence comprise entre 400 et 4500 g/mole, plus préférentiellement entre 500 et 3000 g/mole, encore plus préférentiellement entre 700 et 2800 g/mole, et encore plus préférentiellement comprise entre 1000 et 2600 g/mole.

La présente invention concerne donc l'utilisation de ces maltodextrines branchées pour améliorer la flore du colon du nouveau-né et/ou de l'enfant en bas âge, et ainsi traiter ou prévenir les coliques, les entérocolites et améliorer et/ou favoriser de manière générale le bien-être intestinal du nourrisson et/ou de l'enfant en bas âge, caractérisée en ce que les maltodextrines branchées présentent toutes les caractéristiques énoncées ci-dessus.

En particulier, on pourra utiliser des MDB présentant entre 15 et 35% de liaisons glucosidiques 1-6, une teneur en sucres réducteurs inférieure à 20%, une masse moléculaire moyenne en nombre Mn comprise entre 250 et 4500 g/mole et une masse moléculaire moyenne en poids Mw comprise entre 4000 et 6000 g/mole. Selon une variante, ledit polysaccharide présente un poids moléculaire Mw compris entre 1000 et 6000 g/mole, préférentiellement compris entre 1500 et 5000 g/mole, et plus préférentiellement compris entre 3000 et 5000 g/mole.

Certaines sous-familles de MDB décrites dans la susdite demande peuvent aussi être utilisées conformément à l'invention. Il s'agit par exemple de MDB de hauts poids moléculaires présentant une teneur en sucres réducteurs au plus égale à 5 et un Mn compris entre 2000 et 4500 g/mole. Les MDB de bas poids moléculaires présentant une teneur en sucres réducteurs comprise entre 5 et 20% et une masse moléculaire Mn inférieure à 2000 g/mole peuvent également être employées.

Dans un autre mode de réalisation avantageux de la présente invention, on pourra également utiliser, conformément à l'invention, les maltodextrines hyperbranchées hypo-glycémiantes décrites dans la demande FR 1251810 dont la Demanderesse est titulaire.

Les maltodextrines branchées utilisées conformément à l'invention sont solubles dans l'eau.

Au sens de la présente invention, on entend donc par MDB des maltodextrines dont la teneur en liaisons glucosidiques 1-6 est supérieure à celle des maltodextrines standards. Ainsi, les maltodextrines standards se définissent comme des mélanges purifiés et concentrés de glucose et de polymères de glucose essentiellement liés en 1-4 avec seulement de 4 à 5 % de liaisons glucosidiques 1-6, de poids moléculaires extrêmement variés, complètement solubles dans l'eau et à faible pouvoir réducteur.

Les maltodextrines standards sont classiquement produites par hydrolyse acide ou enzymatique d'amidon. La classification des maltodextrines standards repose principalement sur la mesure de leur pouvoir réducteur, exprimé classiquement par la notion de Dextrose Equivalent (D.E.). Sur ce point particulier, la définition des maltodextrines reprise dans les Monograph Spécifications du Food Chemical Codex précise que la valeur de D.E. ne doit pas excéder 20. La mesure du D.E. ne donne en fait qu'une idée approximative du Degré de Polymérisation (D.P.) moyen du mélange du glucose et des polymères de glucose constitutifs des maltodextrines standards et donc de leur masse moléculaire moyenne en nombre (Mn). Pour compléter la caractérisation de la distribution des masses moléculaires des maltodextrines standards, la détermination d'un autre paramètre est importante, celui de la masse moléculaire moyenne en poids (Mw).

Dans la pratique, les valeurs de Mn et de Mw se mesurent par différentes techniques. On utilise par exemple une méthode de mesure adaptée aux polymères de glucose, qui repose sur la chromatographie de perméation de gel sur des colonnes de chromatographie étalonnées avec des pullulans de masses moléculaires connues.

Le rapport Mw/Mn est appelé indice de polymolécularité (I.P.) et permet de caractériser globalement la distribution des masses moléculaires d'un mélange polymérique. En règle générale, la répartition en masses moléculaires des maltodextrines standards conduit à des I.P. compris entre 5 et 10.

Selon une variante de la présente invention, les MDB présentent un poids moléculaire Mw compris entre 1000 et 6000 g/mole, préférentiellement compris entre 1500 et 5000 g/mole, et plus préférentiellement compris entre 3000 et 5000 g/mole.

Les MDB de la présente invention présentent un caractère d'indigestibilité qui a pour conséquence de diminuer leur pouvoir calorique, en empêchant leur assimilation au niveau de l'intestin grêle. Elles présentent donc une source de fibres indigestibles bénéfiques pour la fermentation colique et pour l'équilibre intestinal, ce qui en fait d'excellents candidats prébiotiques pour la nutrition infantile.

A titre indicatif, leur taux de fibres solubles est généralement supérieur à 80% sur matière sèche. Leur teneur élevée en liaisons glucosidiques 1-6 leur confère des propriétés prébiotiques tout à fait particulières : il est en effet apparu que les bactéries du caecum et du colon de l'homme et des animaux, telles que les bactéries butyrogènes, lactiques ou propioniques métabolisent des composés hautement branchés. D'autre part, ces MDB favorisent le développement des bactéries bifidogènes au détriment des bactéries indésirables. Il en résulte des propriétés tout à fait bénéfiques pour la santé du consommateur, et plus particulièrement pour la santé du nourrisson et/ou de l'enfant en bas âge.

Enfin, le caractère branché desdites MDB diminue considérablement et avantageusement leur tendance à rétrograder, ce qui permet d'envisager leur utilisation dans des applications ou l'absence de rétrogradation est nécessaire, en particulier lors d'un stockage prolongé en solution aqueuse.

Il est en effet du mérite de la Demanderesse d'avoir découvert que les polysaccharides, et plus particulièrement les maltodextrines branchées pouvaient de façon surprenante remplir le rôle de prébiotiques en permettant de s'affranchir des effets négatifs des autres prébiotiques connus de l'art antérieur et déjà utilisés dans les compositions destinées à la nutrition infantile.

Les MDB de la présente invention présentent un caractère d'indigestibilité qui a pour conséquence de diminuer leur pouvoir calorique, en empêchant leur assimilation au niveau de l'intestin grêle. Elles présentent donc une source de fibres indigestibles bénéfiques pour la fermentation colique et pour l'équilibre intestinal, ce qui en fait d'excellents candidats prébiotiques pour la nutrition infantile.

Grâce aux nombreuses propriétés précitées et aux résultats expérimentaux présentés dans les exemples ci-après, les MDB selon la présente invention présentent un effet amélioré par rapport aux autres prébiotiques connus de l'art antérieur et déjà utilisés dans des compositions infantiles.

Il est effectivement connu que les FOS ne sont pas stables à la stérilisation. Cette instabilité se traduit par une hydrolyse progressive, qui génère une libération de glucose et de fructose et une coloration indésirables. Les FOS restent mal tolérés par l'organisme, s'exprimant par la survenue de diarrhées. Il est connu de l'art antérieur que les principaux prébiotiques utilisés aujourd'hui, i.e. les FOS et les GOS principalement, ont également des effets négatifs. A côté de leur effet sur la flore intestinale, ces FOS et GOS ont également un effet sensible sur le comportement intestinal. Au niveau de l'intestin grêle, ils restent en solution dans le chyme et augmentent la pression osmotique, créant un appel d'eau dans la lumière intestinale. Au niveau du colon, la fermentation bactérienne s'accompagne de la production de gaz et d'acides gras à chaine courte qui sont connus pour influencer la motilité intestinale. Si ces effets sont recherchés chez l'adulte pour améliorer le transit intestinal et éviter les problèmes de constipation crées par notre alimentation moderne trop pauvre en fibres, il n'en est pas de même chez le nourrisson où une consommation élevée de prébiotiques peut créer des symptômes pathologiques : diarrhée, flatulence, coliques, etc...

Les prébiotiques de la présente invention permettent justement d'améliorer ces effets négatifs et de les supprimer totalement.

Un exemple de MDB particulièrement adaptées à la présente invention est l'utilisation de NUTRIOSE®, qui est une gamme complète de fibres solubles, reconnues pour leurs bienfaits, et fabriquées et commercialisées par la Demanderesse. Les produits de la gamme NUTRIOSE® sont des dérivés d'amidon de blé ou de maïs partiellement hydrolysés, qui contiennent jusqu'à 85 % de fibre. Cette richesse en fibre permet d'augmenter la fermentation colique, d'améliorer la gestion de calorie, de prolonger le dégagement d'énergie et d'obtenir un taux de sucre inférieur. De plus, la gamme NUTRIOSE® est l'une des fibres les mieux tolérées disponibles sur le marché. Elle montre une tolérance digestive plus élevée, permettant une meilleure incorporation que d'autres fibres, ce qui représente de vrais avantages alimentaires.

Les MDB spécifiques utilisées dans la présente invention répondent en tous points aux critères des prébiotiques. Elles sont en effet considérées comme des ingrédients sélectivement fermentés entrainant des changements spécifiques dans la composition et/ou l'activité de la microflore gastro-intestinale et exercent ainsi des bienfaits sur la santé de l'hôte. Un premier effet positif qu'exercent les prébiotiques, et plus particulièrement les MDB de la présente invention est leur faculté de moduler la flore intestinale du nourrisson et/ou de l'enfant en bas âge.

En effet, il ressort d'études menées par la Demanderesse que la consommation de ces MDB permet d'augmenter le nombre total de Bifidobactéries, de façon dose-dépendante. Cet effet est démontré notamment dans l'exemple 1 ci-après, où il ressort clairement des études menées, que la supplémentation pendant 44 jours d'une alimentation conventionnelle avec 4% de maltodextrines branchées permet d'augmenter d'environ 78% le taux de Bifidobactéries présent dans les fèces chez le porcelet.

D'autres études menées par la Demanderesse permettent de démontrer que les MDB ont un effet stimulateur sur les Bifidobactéries, et plus particulièrement sur les bactéries lactiques acides de type *Lactobacillus paracasei.* Ceci est démontré dans l'exemple 2 de la présente invention.

L'exemple 2 permet également de démontrer que les MDB selon la présente invention permettent également d'inhiber les bactéries potentiellement pathogènes, responsables des problèmes digestifs discutés ci-dessus. En effet, les MDB de l'invention ont un effet inhibiteur supérieur à l'effet inhibiteur d'autres fibres déjà connues comme les GOS ou les FOS. Ainsi sur la croissance de *Clostridium difficile, Clostridium perfringens* et *Escherichia coli,* l'effet inhibiteur des MDB est bien supérieur à l'effet inhibiteur des autres fibres connues de l'art antérieur.

Selon l'invention les MDB ont un réel effet sur l'inhibition des bactéries responsables de coliques et des entérocolites.

La présente invention concerne également l'utilisation de MDB dans une composition pour la nutrition infantile, c'est-à-dire dans une composition ou préparation alimentaire destinée à l'alimentation du nourrisson et/ou de l'enfant en bas âge.

Dans un mode préféré de l'invention, l'utilisation desdites MDB dans une composition pour la nutrition infantile est caractérisée en ce que la composition ou préparation alimentaire comprend de 0,1 à 45%, de préférence de 1 à 20% et plus préférentiellement encore de 1 à 10% en poids sec de maltodextrines branchées.

Selon la présente invention, les MDB peuvent être utilisées seules dans des compositions ou préparations alimentaires destinées à l'alimentation du nourrisson et/ou de l'enfant en bas âge, mais peuvent également être combinées à d'autres fibres connues de l'art antérieur.

Ainsi, selon un mode préférentiel de l'invention, l'utilisation de MDB dans une composition pour la nutrition infantile est caractérisée en ce que ladite composition comprend de 0,1 à 30% d'une autre fibre choisie parmi les Fructo-oligosaccharides (FOS), l'inuline, les Gluco-oligosaccharides (GOS), les Isomalto-oligosaccharides (IMOs), les Trans-galacto-oligosaccharide (TOS) et leurs mélanges quelconques.

Selon un mode particulièrement préféré, l'utilisation de MDB dans une composition pour la nutrition infantile est caractérisée en ce que ladite composition comprend de 0,1 à 45%, de préférence de 1 à 20% et plus préférentiellement encore de 1 à 10% en poids sec de maltodextrines branchées et de 0,1 à 30% d'une autre fibre choisie parmi les Fructo-oligosaccharides (FOS), l'inuline, les Gluco-oligosaccharides (GOS), les Isomalto-oligosaccharides (IMOs), les Trans-galacto-oligosaccharide (TOS) et leurs mélanges quelconques.

Selon la présente invention, les prébiotiques, et plus particulièrement les MDB de la composition ou préparation pour la nutrition infantile jouent également un rôle important dans la modulation du pH des selles du nourrisson et/ou de l'enfant en bas âge. En effet, la dégradation des MDB par la flore colique engendre la formation d'acides gras à chaîne courte, qui acidifient le milieu intestinal.

Ainsi, la présente invention concerne le domaine des prébiotiques dans la nutrition infantile, et plus particulièrement dans la nutrition des enfants de moins de trois ans. La présente invention est par conséquent destinée tout d'abord aux nourrissons pendant les premiers mois de leur vie, lorsque se fait la colonisation du tube digestif. Mais elle concerne également le jeune enfant, victime lui aussi de problèmes digestifs, voire de maladies plus graves, jusqu'à l'introduction dans son alimentation du lait de vache.

La présente invention s'adresse donc à la fois aux nourrissons et jeunes enfants sains mais également à ceux souffrant d'un dysfonctionnement plus ou moins sérieux de leur appareil digestif.

Ainsi, la présente invention concerne l'utilisation de MDB dans une composition pour la nutrition infantile, c'est-à-dire dans une composition ou préparation alimentaire destinée à l'alimentation du nourrisson et/ou de l'enfant en bas âge pour traiter ou prévenir les coliques, les entérocolites et améliorer et/ou favoriser le bien-être intestinal chez le nourrisson et/ou le jeune enfant.

Dans la présente invention, on comprend par utilisation de MDB dans une composition ou préparation alimentaire, une utilisation de MDB dans tout aliment destiné à être donné tel quel, c'est-à-dire sous une forme prête à l'emploi, ou sous une forme nécessitant une dilution préalable dans un support liquide ou solide consommable.

Dans un mode préféré de l'invention, l'utilisation de MDB dans une composition ou préparation est caractérisée en ce que ladite composition se présente sous la forme prête à l'emploi, c'est-à-dire sous la forme de soupe, compote, yaourt, céréales, jus de fruits, thé, biscuits ou tout autre aliment pouvant être consommé par un enfant, aliment donné au moment de la diversification alimentaire.

Dans un autre mode préféré de la présente invention, l'utilisation de MDB dans une composition ou préparation est caractérisée en ce que ladite composition ou préparation se présente sous la forme d'une poudre nécessitant sa mise en solution dans tout liquide potable et consommable par l'enfant. Il peut s'agir entre autre d'une poudre destinée à la reconstitution d'un lait infantile.

Dans un autre mode préféré, l'utilisation de MDB dans une composition ou préparation est caractérisée en ce que ladite composition ou préparation alimentaire se présente sous la forme de céréales. Effectivement, à partir de 6/9 mois, les besoins énergétiques des bébés augmentent. Les céréales jouent progressivement un rôle en tant que complément nutritionnel du lait infantile, qui reste jusqu'à 36 mois un pilier essentiel de l'alimentation du bébé.

La présente invention concerne également l'utilisation de MDB pour la réalisation de préparations initiales pour nourrisson. Ces dites préparations initiales sont destinées à l'alimentation des nourrissons dès la naissance. Elles doivent répondre à toutes les exigences alimentaires du nourrisson pendant les six premiers mois de la vie. Cependant, les préparations initiales pour nourrissons peuvent également être utilisées, en combinaison avec des aliments de complément, au-delà du septième mois et pendant toute la première année de vie.

La présente invention concerne également l'utilisation de MDB pour la réalisation de préparations pour le nourrisson à partir de six mois, dénommés laits de suite.

Dans un autre mode de réalisation, la présente invention concerne également l'utilisation de MDB pour la réalisation de préparations pour le nourrisson à partir de six mois, dénommés laits de croissance. Ces laits sont consommés par des nourrissons âgés d'environ dix mois environ jusqu'à trois ans. En effet, de dix mois à trois ans, les enfants ont encore des besoins nutritionnels spécifiques. A cet âge, le lait infantile est plus adapté que le lait de vache.

Les compositions ou préparation pour la nutrition infantile selon la présente invention et contenant lesdites MDB sont de préférence destinées à l'alimentation des nourrissons.

On admet dans la présente invention que les préparations pour nourrissons sont les seules denrées alimentaires résultant d'une transformation qui répondent totalement aux besoins nutritionnels des nourrissons au cours des premiers mois de leur vie jusqu'à l'introduction d'une alimentation complémentaire appropriée.

Les préparations pour nourrissons décrites dans la présente invention sont donc destinées à l'alimentation de nourrissons dès la naissance. Elles doivent donc répondre à toutes les exigences alimentaires du nourrisson pendant les six premiers mois de vie.

Ces préparations sont fabriquées dans la plupart des cas à base de protéines de lait de vache et contiennent, comme sources d'hydrates de carbone, uniquement du lactose ou du lactose combiné avec d'autres hydrates de carbone.

Afin de se rapprocher le plus possible de la composition du lait maternel, l'utilisation de MDB dans une composition pour la nutrition infantile selon l'invention est caractérisée en ce que ladite composition peut également contenir d'autres éléments. Par exemple, des acides gras essentiels peuvent également être ajoutés, comme l'acide linoléïque, l'acide alpha-linolénique mais aussi l'acide arachidonique et docosahexaénoïque. Ces acides gras ont en effet été identifiés comme ayant un rôle dans le développement cérébral, celui de la vision et du développement psychomoteur.

Les acides gras sont des composés lipidiques qui participent de façon essentielle à la construction et à la vie des cellules. Les acides gras essentiels sont les acides gras que l'organisme n'est pas capable de fabriquer et qu'il doit impérativement trouver dans l'alimentation ou les compléments alimentaires.

D'une manière générale les acides gras et les acides gras essentiels jouent un rôle primordial dans le bon fonctionnement des cellules, notamment pour la composition de leur membrane et l'apport d'énergie. Ils ont également un rôle sur l'inflammation, l'immunité et la coagulation du sang.

Il existe trois types d'acides gras : les acides gras saturés, monoinsaturés et polyinsaturés. La surconsommation d'acides gras saturés est déconseillée, et ne doit en aucun cas excéder 30% des AG totaux consommés, car une surconsommation de ce type d'acides gras est directement corrélée au taux de cholestérol sanguin, et donc à l'augmentation des risques de maladies cardiovasculaires.

Selon un autre mode encore plus préférentiel de la présente invention, l'utilisation de MDB dans une composition pour la nutrition infantile est caractérisée en ce que ladite composition peut également contient au moins un acide gras essentiel.

Les acides gras essentiels sont répartis en deux groupes : le groupe d'acides gras oméga-3 et le groupe d'acides gras oméga-6. Ces deux groupes sont des acides gras polyinsaturés.

Les principaux acides gras du groupe oméga-3 sont l'acide alpha-linolénique, l'acide éicosapentaénoïque et l'acide docosahexaénoïque. On les trouve habituellement dans les algues, les poissons dits gras (maquereau, saumon, thon, sardine, hareng, flétan, anchois...), et dans certaines huiles végétales comme par exemple l'huile de lin, l'huile de colza, l'huile de noix. De très nombreuses études ont démontré les effets positifs d'une alimentation riche en oméga-3 dans l'amélioration de la santé en général et dans la santé cardiovasculaire en particulier.

Les principaux acides gras du groupe oméga-6 sont l'acide linoléique que l'on trouve habituellement dans les huiles végétales comme par exemple l'huile de tournesol, de pépins de raisin, d'onagre, de germe de blé, de noix ; l'acide arachidonique retrouvé dans les chairs d'animaux (viandes) et l'acide docosapentaènoïque retrouvés dans certains poissons et abats.

Selon un mode préféré, l'utilisation de MDB dans une composition pour la nutrition infantile est caractérisée en ce que ladite composition contient au moins un acide gras essentiel choisi dans le groupe constitué par l'acide alpha-linolénique, l'acide linoléique, l'acide arachidonique, l'acide docosapentaènoïque et leur mélange quelconques.

La faible minéralisation du lait de femme et sa richesse en vitamines A, E, C, folates et B12, ainsi qu'en oligo-éléments hormis le fer avec une biodisponibilité inégalée, renforce l'importance de ce côté nutritionnel. Tous ces éléments sont en outre adaptés à l'immaturité digestive, enzymatique et surtout rénale des premiers mois de la vie.

L'objectif de l'utilisation de MDB dans une composition pour la nutrition infantile est caractérisée en ce que ladite composition se rapproche le plus possible, sur le plan nutritionnel, de la composition du lait de femme d'une part, et couvre le mieux possible les besoins énergétiques, minéraux et vitaminiques de l'enfant de l'autre part.

Dans un mode préférentiel, l'utilisation de MDB dans une composition pour la nutrition infantile est caractérisée en ce que ladite composition pour la nutrition infantile contient en outre au moins un élément sélectionné dans le groupe comprenant les acides gras, les vitamines, les oligo-éléments, les sels minéraux et leurs mélanges. Ces éléments sont indispensables au bon développement de l'enfant.

Les vitamines sont des substances qui n'apportent pas d'énergie mais qui sont indispensables au bon fonctionnement de l'organisme. Elles interviennent en faible concentration dans de nombreux processus vitaux. Pour se développer harmonieusement, notre corps doit disposer d'un apport régulier de ces substances qu'il ne peut synthétiser lui même (sauf la vitamine D synthétisée au niveau de la peau sous l'action du soleil, et les Vitamines B2 et K synthétisées chez l'homme par l'intermédiaire de sa flore intestinale, mais cette production endogène ne suffit pas à satisfaire ses besoins, l'obligeant à trouver le complément dans son alimentation).

Elles sont apportées par l'alimentation. Elles sont au nombre de treize et se répartissent en deux catégories :
- les vitamines liposolubles qui sont absorbées en même temps que les graisses et stockées. Elles sont solubles dans les solvants organiques. Ce sont les vitamines A, D, E et K.
- les vitamines hydrosolubles qui ne sont pas stockées de manière prolongée et qui sont excrétées dans les urines quand leur apport est excédentaire. Elles sont solubles dans l'eau. Il s'agit des vitamines C, B1 ou thiamine, B2 ou riboflavine, B3 ou niacine, B5 ou acide pantothénique, B6, B8 ou biotine, B9 ou acide folique, B12.

Selon un autre mode de réalisation avantageux de la présente invention, l'utilisation de MDB dans une composition pour la nutrition infantile est caractérisée en ce que ladite composition pour la nutrition infantile peut également être supplémentée en oligo-éléments. Les oligo-éléments sont une classe de micronutriments nécessaires à la vie mais en quantités très faibles, de l'ordre du µg, et que l'organisme ne peut pas produire.

Les carences, tout comme les excès, sont préjudiciables et toxiques pour l'organisme. L'effet d'un oligo-élément dépend de la dose d'apport. Lorsque l'oligo-élément est dit essentiel, l'absence, comme un apport excessif, sont létaux.

Les oligo-éléments essentiels répondent aux critères suivants :
- être présents à une concentration peu variable dans les tissus d'un organisme ;
- provoquer, par leur absence, des anomalies structurelles et physiologiques proches, et ce de façon similaire dans plusieurs espèces ;
- prévenir ou corriger ces troubles par leur seule présence.

D'un point de vue nutritionnel, il est possible de distinguer deux types d'oligo-éléments selon le risque de carence :
- oligo-éléments essentiels à risque de carence démontré : Iode, Fer, Cuivre, Fluor, Zinc, Sélénium, Chrome, Molybdène,
- oligo-éléments essentiels à faible risque de carence ou non prouvée chez l'Homme : Manganèse, Silicium, Vanadium, Nickel et Etain.

Selon un mode encore plus préférentiel, l'utilisation de MDB dans une composition pour la nutrition infantile est caractérisée en ce que ladite composition pour la nutrition infantile contient au moins un oligo-élément dit essentiel choisi dans le groupe constitué par : l'iode, le fer, le cuivre, le fluor, le zinc, le sélénium et leurs mélanges quelconques.

Selon un autre mode de réalisation avantageux de la présente invention, l'utilisation de MDB dans une composition pour la nutrition infantile est caractérisée en ce que ladite composition pour la nutrition infantile peut également être supplémentée en au moins un sel minéral.

Les sels minéraux sont des composants de l'organisme, d'origine minérale. Comme les vitamines, ils ne sont pas une source d'énergie, mais sont néanmoins indispensables à la vie. Ils se présentent sous forme ionique (anions ou cations).

Dans la présente demande, les sels minéraux concernent les composants présents dans l'organisme en quantité importante (quelques grammes). Ils sont également appelés macroéléments, à la différence des oligo-éléments présents en faible quantité, voire à l'état de trace, qui ont déjà été évoqués dans la présente demande.

Les sels minéraux dans la présente demande sont choisis parmi le groupe constitué par le sodium, le potassium, le calcium, les chlorures, le magnésium, le phosphore, pris seuls ou en combinaison.

Selon un mode encore plus préférentiel, l'utilisation de MDB dans une composition pour la nutrition infantile est caractérisée en ce que ladite composition contient du calcium.

Le calcium remplit un rôle essentiel dans la constitution du squelette et des dents, ainsi que dans la coagulation sanguine, l'activité musculaire, les fonctions hormonales, etc..Les apports calciques en provenance de l'alimentation sont indispensables car l'organisme élimine chaque jour une partie du calcium qu'il contient.

Un des intérêts majeurs de la présente invention est de pouvoir fournir à l'organisme, en plus des bienfaits au niveau intestinal apporté par les MDB, tout le calcium nécessaire au quotidien et au bon fonctionnement de l'organisme.

Ainsi, l'utilisation de MDB dans une composition pour la nutrition infantile, laquelle a été supplémentée par un élément sélectionné dans le groupe comprenant les acides gras, les vitamines, les oligo-éléments, les sels minéraux et leurs mélanges, permet d'élaborer des préparations pour nourrissons et/ou pour enfants en bas âge, dont la composition essentielle répond à leurs besoins nutritionnels.

Selon un autre mode de réalisation préférentiel de la présente invention, l'utilisation de MDB dans une composition pour la nutrition infantile est caractérisée en ce que ladite composition pour la nutrition infantile contient également des nucléotides. Un nucléotide est une molécule organique composée d'une nucléobase, d'un pentose et de 1 à 3 groupements phosphates. Certains nucléotides forment la base de l'ADN et de l'ARN, d'autres sont des cofacteurs ou coenzymes.

Selon un mode préférentiel, le nucléotide est choisi parmi le monophosphate 5' de cytidine, le monophosphate 5' d'uridine, le monophosphate 5' d'adénosine, le monophosphate 5' de guanosine, le monophosphate 5' d'inosine et leurs mélanges quelconques.

Enfin dans un autre mode de réalisation particulièrement avantageux de la présente invention, l'utilisation de MDB dans une composition pour la nutrition infantile est caractérisée en ce que ladite composition comprend également des micro-organismes vivants.

Les micro-organismes en question sont des micro-organismes probiotiques choisis parmi les bactéries ou levures des genres Bifidobacterium, *Lactobacillus, Lactococcus, Streptococcus, Pediococcus, Enterococcus, Propionibacterium, Saccharomyces, Kluyveromyces* et leurs mélanges quelconques.

Selon un autre mode préférentiel, les micro-organismes sont choisis parmi les espèces *Bifidobacterium longum, Lactobacillus casei, Lactobacillus rhamnosus, Lactobacilus helveticus* et *Saccharomyces cerevisiae.*

Selon un mode de réalisation, la composition comprend une quantité de micro-organismes vivants d'au moins 10⁵, de préférence 10⁷, idéalement d'au moins 5x10⁷ cfu/g.

Une des variantes de la présente invention concerne également l'utilisation de MDB dans une composition pour la nutrition infantile caractérisée en ce que ladite composition peut être colorée et/ou aromatisée.

Dans la présente invention, la coloration peut être apportée par un colorant à usage alimentaire, considéré comme un additif alimentaire selon la Directive Européenne N° 89/107/CEE du 21 décembre 1988 relative au rapprochement des législations des États membres concernant les additifs pouvant être employés dans les denrées destinées à l'alimentation humaine.

Dans la présente invention, l'appellation «colorant» désigne toute substance habituellement non consommée comme aliment en soi et habituellement non utilisée comme ingrédient caractéristique dans l'alimentation, possédant ou non une valeur nutritive, et dont l'adjonction intentionnelle aux denrées alimentaires, dans un but technologique au stade de leur fabrication, transformation, préparation, traitement, conditionnement, transport ou entreposage, a pour effet, ou peut raisonnablement être estimée avoir pour effet, qu'elle devient elle-même, ou que ses dérivés deviennent, directement ou indirectement, un composant des denrées alimentaires.

Dans la présente invention, l'appellation «colorant» désigne toute substance ajoutée artificiellement à un aliment pour en changer la couleur, et le rendre théoriquement plus appétissant. Son origine peut être naturelle (organique ou minérale) ou de synthèse. Des exemples de colorants alimentaires susceptibles d'être ajoutés à la composition ou préparation pour la nutrition infantile sont par exemple la curcumine (jaune), le rouge de Cochenille A (rouge), les chlorophyllines (vert), le caramel (brun), les caroténoïdes (orange),...

D'autres exemples de colorants utilisables dans la présente invention sont également les pigments naturels comme par exemple les pigments naturels de carotte, de langouste, de poisson, ainsi que les pigments naturels de fleurs de feuilles et de fruits (abricots, fruits rouges,..).

Selon la présente invention, l'utilisation de MDB dans une composition pour la nutrition infantile est caractérisée en ce que ladite composition peut également être aromatisée grâce à l'ajout d'un ou plusieurs arômes.

Dans la présente invention, l'appellation « arôme » désigne toutes les substances non destinées à être consommées en l'état, qui sont ajoutées aux denrées alimentaires pour leur conférer une odeur et/ou un goût ou modifier ceux-ci. Elles sont issues ou constituées des catégories suivantes: substances aromatisantes, préparations aromatisantes, arômes obtenus par traitement thermique, arômes de fumée, précurseurs d'arôme ou autres arômes ou leurs mélanges.

Les substances aromatisantes sont des substances chimiques définies, ce qui inclut les substances aromatisantes obtenues par synthèse chimique ou isolées par des procédés chimiques, et les substances aromatisantes naturelles. Les préparations aromatisantes sont des arômes autres que des substances chimiques définies, qui sont obtenues par des procédés physiques, enzymatiques ou microbiologiques appropriés, à partir de matières d'origine végétale, animale ou microbiologique prises en l'état ou après leur transformation pour la consommation humaine. Les précurseurs d'arôme tels que les hydrates de carbone, les oligopeptides et les acides aminés confèrent une flaveur aux denrées alimentaires par des réactions chimiques qui se produisent pendant la transformation de ces denrées.

Une autre variante de la présente invention concerne également une composition pour la nutrition infantile pour traiter ou prévenir les coliques, les entérocolites et améliorer et/ou favoriser de manière générale le bien-être intestinal comprenant des maltodextrines branchées, caractérisée en ce que les maltodextrines branchées présentent :
- entre 15 et 50%, préférentiellement entre 22% et 45%, plus préférentiellement entre 20% et 40%, et encore plus préférentiellement entre 25% et 35%, de liaisons glucosidiques 1-6,
- une teneur en sucres réducteurs inférieure à 20%, préférentiellement comprise entre 2% et 20%, plus préférentiellement entre 2,5% et 15%, et encore plus préférentiellement entre 3,5% et 10%,
- un indice de polymolécularité inférieur à 5, préférentiellement compris entre 1 et 4, plus préférentiellement entre 1,5 et 3, et
- une masse moléculaire moyenne en nombre Mn inférieure à 4500 g/mole, préférentiellement comprise entre 400 et 4500 g/mole, plus préférentiellement entre 500 et 3000 g/mole, encore plus préférentiellement entre 700 et 2800 g/mole, et encore plus préférentiellement comprise entre 1000 et 2600 g/mole.

Dans un mode préféré de l'invention, ladite composition pour la nutrition infantile comprend de 0,1 à 45%, de préférence de 1 à 20% et plus préférentiellement encore de 1 à 10% en poids sec de maltodextrines branchées.

Dans un autre mode préféré de l'invention, ladite composition pour la nutrition infantile comprend de 0,1 à 45%, de préférence de 1 à 30% et plus préférentiellement encore de 1 à 20% en poids de maltodextrines branchées sur le poids total de la composition ou préparation alimentaire prête à être consommée.

La présente invention s'adresse donc à la fois aux nourrissons et jeunes enfants sains mais également à ceux souffrant d'un dysfonctionnement plus ou moins sérieux de leur appareil digestif.

Ainsi, la présente invention concerne une composition pour la nutrition infantile comprenant au moins des maltodextrines branchées pour traiter ou prévenir les coliques, les entérocolites et améliorer et/ou favoriser le bien-être intestinal chez le nourrisson et/ou le jeune enfant.

Dans la présente invention, on comprend par composition ou préparation alimentaire, tout aliment destiné à être donné tel quel, c'est-à-dire sous une forme prête à l'emploi, ou sous une forme nécessitant une dilution préalable dans un support liquide ou solide consommable.

Dans un mode préféré de l'invention, ladite composition ou préparation se présente sous la forme prête à l'emploi, c'est-à-dire sous la forme de soupe, compote, yaourt, céréales, jus de fruits, thé, biscuits ou tout autre aliment pouvant être consommé par un enfant, aliment donné au moment de la diversification alimentaire.

Dans un autre mode préféré de la présente invention, ladite composition ou préparation se présente sous la forme d'une poudre nécessitant sa mise en solution dans tout liquide potable et consommable par l'enfant. Il peut s'agir entre autre d'une poudre destinée à la reconstitution d'un lait infantile.

Dans un dernier mode préféré de la présente invention, ladite composition ou préparation contient également un élément choisi dans le groupe comprenant le calcium, le phosphore, la vitamine B1, la vitamine B2, la vitamine B12, la vitamine B19, la vitamine A, la vitamine D et leurs mélanges, les acides gras essentiels.

La présente invention a également pour objet une poudre granulée comprenant des maltodextrines branchées, caractérisée en ce qu'elle présente un diamètre moyen volumique laser D4,3, compris entre 10µm et 500µm, de préférence entre 50µm et 350µm et encore plus préférentiellement entre 70µm et 250µm, et une matière sèche, déterminée après étuvage à 130°C pendant 2 heures, supérieure à 80%, de préférence supérieure à 85%, et encore plus préférentiellement supérieure à 90%.

Dans un mode préféré de la présente invention, la poudre granulée est mise en suspension dans tout liquide potable destiné à la consommation humaine.

Ainsi, la présente invention concerne également une composition liquide obtenue par dissolution de la poudre granulée comprenant les maltodextrines branchées de la présente invention, caractérisé en ce que le taux de dissolution de la poudre granulée dans le liquide est compris entre 2 et 30% en poids sec, de préférence entre 2 et 20%, plus préférentiellement encore entre 3 et 15%, et en particulier entre 5 et 10%.

De préférence, la poudre granulée est mise en solution dans un liquide choisi dans le groupe constitué par l'eau, les jus de fruits, les nectars de fruits, les jus de légumes, les nectars de légumes.

De manière encore plus préférentielle, la poudre granulée est mise en solution dans de l'eau, ladite eau pouvant être de l'eau de source, de l'eau minérale, gazéifiée naturellement ou par adjonction de gaz carbonique ou non gazéifiée.

Dans un mode préférentiel de l'invention, la poudre granulée ou la composition liquide obtenue par dissolution de la poudre granulée selon l'invention est utilisée comme substitut de lait d'origine animal, et plus particulièrement de lait de vache.

Dans un autre mode préférentiel de l'invention, la poudre granulée ou la composition liquide obtenue par dissolution de la poudre granulée selon l'invention est utilisée dans la préparation des aliments d'allaitement, et plus particulièrement dans la préparation de laits d'allaitements pour les nourrissons. Selon le liquide choisi pour la mise en solution de la poudre granulée, le pH peut varier. Eventuellement il peut être corrigé par tout moyen connu par l'homme du métier, et notamment par l'utilisation d'acides ou de bases à usage alimentaire. Dans un autre mode encore plus préférentiel, la poudre granulée ou la composition liquide obtenue par dissolution de la poudre granulée selon l'invention peut être supplémentée avec d'autres éléments, afin de satisfaire et de répondre à toutes les caractéristiques organoleptiques et nutritionnelles ciblées.

L'invention sera encore mieux comprise à la lecture des exemples qui suivent, lesquels se veulent illustratifs, en faisant seulement état de certains modes de réalisation et de certaines propriétés avantageuses selon l'invention, et non limitatifs.

### EXEMPLES

Tous les exemples ci-dessous ont été réalisés avec du NUTRIOSE® FB06, commercialisé par la société Demanderesse, pouvant être obtenue à partir de blé ou de maïs et caractérisée par un taux de fibres de 85% en poids sec et contenant au maximum 0,5% en poids sec de DP1-DP2.

### EXEMPLE 1 : Effets du NUTRIOSE® sur le taux de Bifidobactéries fécales chez le porcelet

L'objectif de cet essai est d'étudier les effets du NUTRIOSE® FB06 sur le taux de Bifidobactéries fécales chez le porcelet.

L'essai est effectué sur 2 groupes de 8 porcelets sevrés d'un poids de 6.53 ± 0,44 kg au début de l'étude.

Les traitements expérimentaux sont les suivants :
Lot n°1: animaux témoins nourris à l'aide d'une alimentation conventionnelle contenant 4% de dextrose, comme produit de référence,
Lot n°2 : animaux nourris à l'aide d'une alimentation conventionnelle contenant 4% de NUTRIOSE® FB06.

Après 44 jours de supplémentation avec l'un des deux régimes, les fèces des animaux des 2 groupes sont recueillies et congelées à -80 °C. Une analyse par PCR en temps réel permet de déterminer les taux de Bifidobactéries par la quantification de l'ARNr 16S.

Le tableau 1 suivant présente les résultats obtenus.

**Tableau 1: résultats**

| | Lot n°1 | Lot n°2 | Valeur de p |
|---|---|---|---|
| *Bifidobactérium* spp. (log 10 copie gene 16S/g) +/- erreur standard | 2.39+/-0.51 | 4.27+/-0.59 | 0.034 |

Le lot n°2 de porcelets nourris avec une alimentation conventionnelle contenant 4% de maltodextrines branchées de type NUTRIOSE® FB06 possède un taux de Bifidobactéries plus important d'environ 78% que le lot n°1 de porcelets nourris avec une alimentation conventionnelle exempte de maltodextrines branchées.

Les résultats montrent que la consommation de NUTRIOSE® FB06 pendant 44 jours permet d'augmenter significativement la population de Bifidobactéries chez le porcelet.

### EXEMPLE 2 : Effets in-vitro de différents prébiotiques sur la flore digestive

L'objectif de cet essai *in-vitro,* réalisé dans des microplaques, est de mesurer l'effet de différents produits sur la stimulation ou l'inhibition de la croissance de bactéries pouvant potentiellement coloniser le côlon du nouveau né.

Les 4 produits testés à 50mg/ml sont le NUTRIOSE® FB06, un FOS (Actilight 950P), un GOS (Purimune High Purity) ainsi que le mélange FOS/GOS (1:9). Les solutions obtenues sont filtrées sur Millipore 0,22 microns pour éviter toute contamination. Le contrôle négatif est l'eau.

Les préparations bactériennes sont calibrées à 10⁴-10⁵ UFC/ml.

Les milieux utilisés, les conditions de culture et les bactéries testées sont listés dans le tableau 1. La croissance de chacune des bactéries est suivie au cours du temps en présence ou en l'absence du produit à tester avec un lecteur microplaque. La lecture est réalisée à une densité optique de 595nm toutes les heures.

**Tableau 2 : souches bactériennes utilisées, conditions de culture**

| Souches CIP: Collection Institut Pasteur | Milieux de culture | Conditions de culture |
|---|---|---|
| Lactobacillus rhamnosus ATCC 53.103 | Bouillon Man Rogosa Sharp (MRS) | 37 °C, aérobiose |
| Lactobacillus paracaseï subsp paracaseï CIP 103.704 | Bouillon Man Rogosa Sharp (MRS) | 37°C, 5% CO₂ |
| Lactobacillus plantarum CIP A159 | Bouillon Man Rogosa Sharp (MRS) | 37°C, 5% CO₂ |
| Bacteroides fragilis CIP 105.892 | Bouillon anaérobie CIP | 37 °C, anaérobiose |
| Bacteroides ovatus CIP 103.756 | Bouillon anaérobie CIP | 37 °C, anaérobiose |
| Bacteroides thetaiotaomicron CIP 104.207 | Bouillon anaérobie CIP | 37 °C, anaérobiose |
| Clostridium difficile CIP 104.282 | Bouillon anaérobie CIP | 37 °C, anaérobiose |
| Clostridium perfringens ATCC 13.124 | Bouillon renforced clostridia medium (RCM) | 37 °C, anaérobiose |
| Escherichia coli CIP 53.126 | TS | 37 °C, aérobiose |
| Campylobacter jejuni ATCC 29.428 | Columbia+sang de mouton, Columbia+serum de cheval | 37 °C, microaérophilie |
| Enterococcus faecalis CIP 103.214 | Bouillon coeur - cervelle | 37 °C, aérobiose |
| Enterococcus faecium CIP 102.379 | Bouillon coeur - cervelle | 37 °C, aérobiose |
| Klebsiella oxytoca ATCC 49.131 | TS | 37 °C, aérobiose |
| Staphylococcus aureus CIP 4.83 | TS | 37 °C, aérobiose |

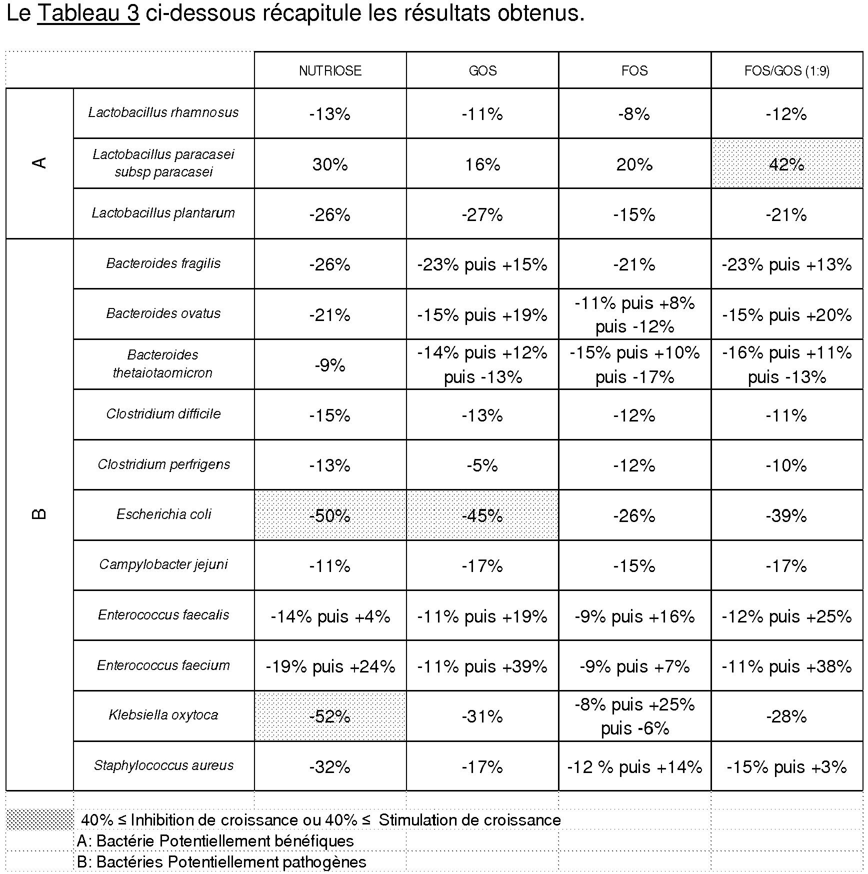

Les résultats montrent :
- une stimulation de la croissance de *Lactobacillus paracasei subsp paracasei* avec les 4 produits et en particulier pour le mélange FOS/GOS,
- une inhibition de la croissance de *Lactobacillus plantarum* et *Lactobacillus rhamnosus* avec les 4 produits et dans des proportions identiques,
- une inhibition de la croissance de *Bacteroides fragilis, Bacteroides ovatus, Bacteroides thetaiotaomicron, Clostridium difficile, Clostridium perfringens, Campylobacter jejuni, Enterococcus faecium,* dans des proportions identiques pour les 4 produits,
- une inhibition de la croissance de *Klebsiella oxytoca* et *Staphylococcus aureus* plus particulièrement marquée avec le NUTRIOSE® FB06,
- une inhibition de la croissance d'*Escherichia coli* plus particulièrement marquée avec le NUTRIOSE® FB06 et le GOS.

### EXEMPLE 3

### Objectif de l'essai

L'objectif de cet essai *in-vivo* est d'étudier les effets des maltodextrines branchées du type NUTRIOSE® FB06 chez la souris axénique inoculée par une souche entérotoxinogène de *Clostridium perfringens* sur l'induction de la mortalité des animaux, la régulation de la colonisation de la souche au niveau intestinal et la régulation de la translocation de la souche. Ce type de souche entérotoxinogène est responsable d'entérocolites ulcéronécrosantes. En effet, la survenue de septicémies à gram négatif entraîne la mort de nouveau-né puisque ce type de souche s'implante au niveau du côlon des enfants et transloque vers les organes périphériques.

### Protocole

8 ou 9 souris par groupe (mâles et femelles) sont mises en expérimentation (Souris C3H/He axéniques de 6 à 8 semaines). Les animaux sont hébergés en isolateur.

La souche de *Clostridium perfingens* est isolée d'une biopsie de patiente souffrant d'entérocolite ulcéronécrosante. L'inoculum est préparé dans un milieu Columbia supplémenté avec du chlorhydrate de cystéine et du glucose. L'inoculation de cette souche s'effectue par gavage oesophagique à J3 de l'essai.

La quantité administrée est de 10⁶UFC/animal.

Les produits à l'essai sont administrés quotidiennement dans la boisson et sont stérilisés par filtration (0,2µ) et autoclavage (110°C, 30 minutes).

Les 4 groupes sont les suivants :
- Eau osmosée (témoin négatif)
- Lactose à 70g/L ; le lactose active les gènes de la sporulation et de la synthèse d'entérotoxines via le galactose.
- NUTRIOSE® FB06 à 4g/L
- NUTRIOSE® FB06 à 35g/L (équivaut à la quantité de glucose du lot lactose 70g/L)

La durée totale de l'essai est de 14 jours.
24 heures après l'implantation de *Clostridium perfringens,* 2 animaux par groupe sont sacrifiés afin de dénombrer la souche dans différents organes.
48 heures après l'implantation de *Clostridium perfringens,* des fèces sont recueillies pour réaliser un dénombrement de *Clostridium perfringens.*
10 jours après l'implantation l'ensemble des animaux est sacrifié afin de dénombrer la souche dans différents organes.

### Résultats - Mortalité des souris

Les résultats montrent que 100% des souris du groupe eau, NUTRIOSE® FB06 à 4g/L, et NUTRIOSE® FB06 à 35g/L sont vivantes.
100% des souris du groupe positif lactose à 70g/L sont mortes entre J+2 et J+3.
Le NUTRIOSE® FB06 n'a donc pas d'effet létal contrairement au lactose. On peut penser qu'il n'y a pas d'induction de la toxinogénèse quelle que soit la concentration testée.

### Résultats - Implantation de Clostridium perfringens dans les fèces (48h post-implantation)

**Tableau 4**

| | Dénombrement (log UFC/g de fèces) 48h post-implantation n=4 souris/groupe |
|---|---|
| Témoin négatif eau | 8.51 ± 0.26 |
| NUTRIOSE® FB 06 à 4g/L | 8.04 ± 0.64 |
| NUTRIOSE® FB 06 à 35g/L | 8.25 ± 0.42 |
| Lactose | Souris mortes ou moribondes |

Le dénombrement de *Clostridium perfringens* dans les fèces est identique entre les groupes eau, NUTRIOSE® FB06 à 4g/L et NUTRIOSE® FB06 à 35g/L.

Ces résultats laissent présager une translocation bactérienne minime puisque l'excrétion fécale de la bactérie est importante.

### Résultats - Colonisation de Clostridium perfringens dans l'appareil digestif et translocation de Clostridium perfringens (24h post-implantation)

2 souris par groupe ont été sacrifiées.
L'iléon, le caecum, le côlon et les plaques de Peyers représentent la partie intestinale tandis que le sang, les reins, la rate, le foie, les poumons et le thymus sont les organes cibles d'une translocation bactérienne.

**Tableau 5 - Dénombrement de Clostridium perfringens dans différents organes**

| Dénombrement (log UFC/g) | Témoin négatif eau | Lactose | NUTRIOSE® FB06 à 4g/L | NUTRIOSE® FB06 à 35g/L |
|---|---|---|---|---|
| Iléon | 7,2 ± 0,5 | 3,4 ± 0,8 | 6,7 ± 0,4 | 7,6 (F) |
| Caecum | 8,4 ± 0,3 | 5,5 ± 0,8 | 7,9 ± 0,3 | 8,0 (F) |
| Côlon | 7,8 ± 0,1 | 6,6 ± 0,1 | 8,5 ± 0,6 | 8,2 (F) |
| Plaques Peyers | 3,5 ± 0,9 | N.D | 3,2 ± 1,0 | 3,9 (F) |
| Sang | N.D | 2,3 (M) | 2 (M) | N.D |
| Rein | N.D | 3,0 (F) | N.D | N.D |
| Rate | N.D | 4,2 (M) | 3,7 (F) | N.D |
| Foie | N.D | 4,4 (M) | 3,5 ± 0,3 | 3,0 (M) |
| Poumon | N.D | N.D | 3,0 (F) | N.D |
| Thymus | N.D | N.D | 3,7 (M) | 3,6 (F) |
| Nombre de souris | 2 | 2 | 2 | 2 |

| | | | | |
|---|---|---|---|---|
| N.D: non détecté, M : souris mâle, F : souris femelle | | | | |

Par rapport aux souris témoins, les souris sous lactose montrent une faible colonisation de l'intestin. Par contre, le lactose favorise la translocation bactérienne, probablement en partie basse, les plaques de Peyer n'étant pas contaminées.

Les souris consommant le NUTRIOSE® FB06 à 4g/L et 35g/L montrent une colonisation intestinale similaire aux témoins, en lien avec les résultats du tableau 4. La translocation bactérienne semble être limitée à la dose de NUTRIOSE® FB06 de 35g/L.

### Résultats - Colonisation de Clostridium perfringens dans l'appareil digestif et translocation de Clostridium perfringens (10 jours post-implantation)

**Tableau 6 - Dénombrement de Clostridium perfringens dans différents organes**

| Dénombrement (log UFC/g) | Témoin négatif eau | NUTRIOSE® FB06 à 4g/L | NUTRIOSE® FB06 à 35g/L |
|---|---|---|---|
| Iléon | 6,1 ± 0,6 (6) | 6,6 ± 0,7 (7) | 6,5 ± 0,6 (6) |
| Caecum | 8,7 ± 0,1 (6) | 8,8 ± 0,3 (7) | 8,5 ± 0,3 (6) |
| Côlon | 8,6 ± 0,5(6) | 8,3 ± 0,4 (7) | 8,5 ± 0,4 (6) |
| Plaques Peyers | 2,5 ± 0,7 (4) | 2,1 ± 0,5 (6) | 2,2 ± 0,7 (6) |
| Sang | 4 (1 M) | N.D | N.D |
| Rein | 2,9 (1F) | 3,1 (1) | N.D |
| Rate | N.D | 3,2(1) | N.D |
| Foie | 2,8 ± 0,4 (2) | N.D | N.D |
| Poumon | N.D | 3,3 (1) | N.D |
| Thymus | N.D | N.D | N.D |
| Nombre de souris | 6 | 7 | 6 |

| | | | |
|---|---|---|---|
| N.D: non détecté, M : souris mâle, F : souris femelle | | | |

**Tableau 7 - Nombre d'organes contaminés par Clostridium perfringens**

| Nombre d'organes contaminés | Témoin négatif eau (n=6) | NUTRIOSE® FB06 à 4g/L (n=7) | NUTRIOSE® FB06 à 35g/L (n=6) |
|---|---|---|---|
| 0 organe | 3 | 4 | 6 |
| 1 organe | 2 | 3 | 0 |
| 2 organes | 1 | 0 | 0 |

Les tableaux 6 et 7 montrent une absence de détection de *Clostridium perfringens* dans le sang, les reins, la rate, le foie, les poumons, le thymus, démontrant une limitation complète de la translocation bactérienne.

Pour conclure, lorsque seul *Clostridium perfringens* colonise l'intestin des souris, les solutions de NUTRIOSE® FB06 ne montrent pas d'effet létal contrairement au lactose. Il n'y a donc pas d'induction de la toxinogénèse quelque soit la concentration de NUTRIOSE® FB06 administrée. En outre, le bilan de translocation montre un effet régulateur dose dépendant. La dose élevée induit lentement la régulation de la dissémination bactérienne.

## Revendications

1. Maltodextrine branchée pour son utilisation pour améliorer la flore du colon du nouveau-né, et ainsi traiter ou prévenir les coliques, les entérocolites, **caractérisée en ce que** ladite maltodextrine branchée présente :
- entre 15 et 50%, préférentiellement entre 22% et 45%, plus préférentiellement entre 20% et 40%, et encore plus préférentiellement entre 25% et 35%, de liaisons glucosidiques 1-6
- une teneur en sucres réducteurs inférieure à 20%, préférentiellement comprise entre 2% et 20%, plus préférentiellement entre 2,5% et 15%, et encore plus préférentiellement entre 3,5% et 10%,
- un indice de polymolécularité inférieur à 5, préférentiellement compris entre 1 et 4, plus préférentiellement entre 1,5 et 3, et
- une masse moléculaire moyenne en nombre Mn inférieure à 4500 g/mole, préférentiellement comprise entre 400 et 4500 g/mole, plus préférentiellement entre 500 et 3000 g/mole, encore plus préférentiellement entre 700 et 2800 g/mole, et encore plus préférentiellement comprise entre 1000 et 2600 g/mole.

2. Maltodextrine branchée pour son utilisation selon la revendication 1, **caractérisée en ce qu'**elle améliore et/ou favorise de manière générale le bien-être intestinal du nourrisson.

3. Préparation alimentaire destinée à l'alimentation des nourrissons pour son utilisation chez le nourrisson pour traiter ou prévenir les coliques, les entérocolites, la dite préparation comprenant des maltodextrines branchées, **caractérisée en ce que** les dites maltodextrines branchées présentent :
- entre 15 et 50%, préférentiellement entre 22% et 45%, plus préférentiellement entre 20% et 40%, et encore plus préférentiellement entre 25% et 35%, de liaisons glucosidiques 1-6,
- une teneur en sucres réducteurs inférieure à 20%, préférentiellement comprise entre 2% et 20%, plus préférentiellement entre 2,5% et 15%, et encore plus préférentiellement entre 3,5% et 10%,
- un indice de polymolécularité inférieur à 5, préférentiellement compris entre 1 et 4, plus préférentiellement entre 1,5 et 3, et
- une masse moléculaire moyenne en nombre Mn inférieure à 4500 g/mole, préférentiellement comprise entre 400 et 4500 g/mole, plus préférentiellement entre 500 et 3000 g/mole, encore plus préférentiellement entre 700 et 2800 g/mole, et encore plus préférentiellement comprise entre 1000 et 2600 g/mole.

4. Préparation alimentaire pour son utilisation selon la revendication 3, **caractérisée en ce qu'**elle comprend de 0,1 à 45%, de préférence de 1 à 30% et plus préférentiellement encore 1 à 20% en poids de maltodextrines branchées sur le poids total ou le volume total de la composition ou préparation alimentaire prête à être consommée.

5. Préparation alimentaire pour son utilisation selon l'une quelconque des revendications 3 ou 4, **caractérisée en ce que** ladite préparation alimentaire comprend de 0,1 à 30% en poids sec d'une autre fibre choisie parmi les Fructo-oligosaccharides (FOS), l'inuline, les Gluco-oligosaccharides (GOS), les Isomalto-oligosaccharides (IMOs), les Trans-galacto-oligosaccharide (TOS) et leurs mélanges quelconques.

6. Préparation alimentaire pour son utilisation selon l'une quelconque des revendications 3 à 5, **caractérisée en ce qu'**elle se présente sous la forme d'une poudre nécessitant sa mise en solution dans tout liquide potable et consommable.

7. Préparation alimentaire pour son utilisation selon l'une quelconque des revendications 3 à 6, **caractérisée en ce que** ladite préparation alimentaire contient en outre au moins un élément sélectionné dans le groupe comprenant les acides gras, les vitamines, les oligo-éléments, les sels minéraux et leurs mélanges.

8. Préparation alimentaire pour son utilisation selon l'une quelconque des revendications 3 à 7, **caractérisée en ce qu'**elle contient un élément choisi dans le groupe comprenant le calcium, le phosphore, la vitamine B1, la vitamine B2, la vitamine B12, la vitamine B19, la vitamine A, la vitamine D et leurs mélanges, et les acides gras essentiels.

9. Préparation alimentaire pour son utilisation selon l'une quelconque des revendications 3 à 8, **caractérisée en ce que** ladite préparation alimentaire est également aromatisée et/ou colorée.

10. Préparation alimentaire pour son utilisation selon l'une quelconque des revendications 3 à 8, **caractérisée en ce qu'**elle améliore et/ou favorise de manière générale le bien-être intestinal du nourrisson.

## Patentansprüche

1. Verzweigtes Maltodextrin zur Verwendung zur Verbesserung der Kolonflora des Neugeborenen und dann zur Behandlung oder zur Prävention von Koliken, Enterokoliken, **dadurch gekennzeichnet, dass** das verzweigte Maltodextrin aufweist:
- zwischen 15 und 50 %, vorteilhafterweise zwischen 22 % und 45 %, stärker bevorzugt zwischen 20 % und 40 % und noch stärker bevorzugt zwischen 25 % und 35 % 1-6 glucosidische Verbindungen,
- einen Gehalt an reduzierenden Zuckern unterhalb von 20 %, vorzugsweise umfasst zwischen 2 % und 20 %, stärker bevorzugt zwischen 2.5 % und 15 % und noch stärker bevorzugt zwischen 3,5 % und 10 %,
- einen Polymolekularitätsindex unterhalb von 5, vorzugsweise umfasst zwischen 1 und 4, stärker bevorzugt zwischen 1,5 und 3, und
- eine molekulare Masse im Zahlenmittel Mn unterhalb von 4500 g/mol, vorzugsweise umfasst zwischen 400 und 4500 g/mol, stärker bevorzugt zwischen 500 und 3000 g/mol, noch stärker bevorzugt zwischen 700 und 2800 g/mol und noch stärker bevorzugt umfasst zwischen 1000 und 2600 g/mol.

2. Verzweigtes Maltodextrin zur Verwendung gemäß Anspruch 1, **dadurch gekennzeichnet, dass** es auf allgemeine Weise das intestinale Wohlbefinden des Kleinkinds verbessert und/oder fördert.

3. Nahrungsformulierung, bestimmt zur Ernährung von Kleinkindern, zur Verwendung beim Kleinkind zur Behandlung oder zur Prävention von Koliken, Enterokoliken, wobei die Formulierung verzweigte Maltodextrine umfasst, **dadurch gekennzeichnet, dass** die verzweigten Maltodextrine aufweisen:
- zwischen 15 und 50 %, vorteilhafterweise zwischen 22 % und 45 %, stärker bevorzugt zwischen 20 % und 40 % und noch stärker bevorzugt zwischen 25 % und 35 % 1-6 glucosidische Verbindungen,
- einen Gehalt an reduzierenden Zuckern unterhalb von 20 %, vorzugsweise umfasst zwischen 2 % und 20 %, stärker bevorzugt zwischen 2.5 % und 15 % und noch stärker bevorzugt zwischen 3,5 % und 10 %,
- einen Polymolekularitätsindex unterhalb von 5, vorzugsweise umfasst zwischen 1 und 4, stärker bevorzugt zwischen 1,5 und 3, und
- eine molekulare Masse im Zahlenmittel Mn unterhalb von 4500 g/mol, vorzugsweise umfasst zwischen 400 und 4500 g/mol, stärker bevorzugt zwischen 500 und 3000 g/mol, noch stärker bevorzugt zwischen 700 und 2800 g/mol und noch stärker bevorzugt umfasst zwischen 1000 und 2600 g/mol.

4. Nahrungsformulierung zur Verwendung gemäß Anspruch 3, **dadurch gekennzeichnet, dass** sie von 0,1 bis 45 %, vorzugsweise von 1 bis 30 % und noch stärker bevorzugt von 1 bis 20 % von verzweigten Maltodextrinen als Gewichtsanteil bezogen auf das Gesamtgewicht oder das Gesamtvolumen der Zusammensetzung oder Nahrungsformulierung umfasst, welche fertig zum Gebrauch ist.

5. Nahrungsformulierung zur Verwendung gemäß einem der Ansprüche 3 oder 4, **dadurch gekennzeichnet, dass** die Nahrungsformulierung von 0,1 bis 30%, bezogen auf das Trockengewicht, eines anderen Ballaststoffes ausgewählt aus Fructo-Oligosacchariden (FOS), Inulin, Gluco-Oligosacchariden (GOS), Isomalto-Oligosacchariden (IMOs), Trans-Galacto-Oligosacchariden (TOS) und ihren Mischungen davon, umfasst.

6. Nahrungsformulierung zur Verwendung gemäß einem der Ansprüche 3 bis 5, **dadurch gekennzeichnet, dass** sie eine Puderform aufweist, welche die Lösung in allen trinkbaren und konsumierbaren Flüssigkeit erfordert.

7. Nahrungsformulierung zur Verwendung gemäß einem der Ansprüche 3 bis 6, **dadurch gekennzeichnet, dass** die Nahrungsformulierung außerdem wenigstens ein Element enthält ausgewählt aus der Gruppe umfassend Fettsäuren, Vitamine, Oligoelemente, Mineralsalze und ihre Mischungen.

8. Nahrungsformulierung zur Verwendung gemäß einem der Ansprüche 3 bis 7, **dadurch gekennzeichnet, dass** sie ein Element enthält ausgewählt aus der Gruppe umfassend Calcium, Phosphor, Vitamin B1, Vitamin B2, Vitamin B12, Vitamin B19, Vitamin A, Vitamin D und ihre Mischungen und essentielle Fettsäuren.

9. Nahrungsformulierung zur Verwendung gemäß einem der Ansprüche 3 bis 8, **dadurch gekennzeichnet, dass** die Nahrungsformulierung gleichmäßig aromatisiert und/oder gefärbt ist.

10. Nahrungsformulierung zur Verwendung gemäß einem der Ansprüche 3 bis 8, **dadurch gekennzeichnet, dass** sie auf allgemeine Weise das intestinale Wohlbefinden des Kleinkinds verbessert und/oder fördert.

## Claims

1. Branched maltodextrin for its use for improving the flora of the colon of newborn babies, and thus treating or preventing colic and enterocolitis **characterized in that** the branched maltodextrin has:
- between 15% and 50%, preferentially between 22% and 45%, more preferentially between 20% and 40%, and even more preferentially between 25% and 35%, of 1-6 glucosidic linkages,
- a reducing sugar content of less than 20%, preferentially between 2% and 20%, more preferentially between 2.5% and 15%, and even more preferentially between 3.5% and 10%,
- a polydispersity index of less than 5, preferentially between 1 and 4, more preferentially between 1.5 and 3, and
- a number-average molecular weight Mn of less than 4500 g/mol, preferentially between 400 and 4500 g/mol, more preferentially between 500 and 3000 g/mol, even more preferentially between 700 and 2800 g/mol, and even more preferentially between 1000 and 2600 g/mol.

2. Branched maltodextrin for its use as claimed in claim 1 for improving and/or generally promoting the intestinal well-being of infants.

3. A food composition for feeding infant nutrition for its use for infants for treating or preventing colic and enterocolitis, said composition comprising branched maltodextrins, **characterized in that** said branched maltodextrins have:
- between 15% and 50%, preferentially between 22% and 45%, more preferentially between 20% and 40%, and even more preferentially between 25% and 35%, of 1-6 glucosidic linkages,
- a reducing sugar content of less than 20%, preferentially between 2% and 20%, more preferentially between 2.5% and 15%, and even more preferentially between 3.5% and 10%,
- a polydispersity index of less than 5, preferentially between 1 and 4, more preferentially between 1.5 and 3, and
- a number-average molecular weight Mn of less than 4500 g/mol, preferentially between 400 and 4500 g/mol, more preferentially between 500 and 3000 g/mol, even more preferentially between 700 and 2800 g/mol, and even more preferentially between 1000 and 2600 g/mol.

4. Food composition for its use as claimed in claim 3, **characterized in that** it comprises from 0.1% to 45%, preferably from 1% to 20% and even more preferentially from 1% to 20% by weight of branched maltodextrins relative to the total weight or total volume of the food composition or formula ready to be consumed.

5. Food composition for its use as claimed in either one of claims 3 and 4, **characterized in that** the food composition comprises from 0.1% to 30% by dry weight of another fiber chosen from fructooligosaccharides (FOSs), inulin, glucooligosaccharides (GOSs), isomaltooligosaccharides (IMOs), transgalactooligosaccharides (TOSs) and any mixtures thereof.

6. Food composition for its use as claimed in any one of claims 3 to 5, **characterized in**, **characterized in that** it is in the form of a powder which needs to be dissolved in any drinkable liquid that can be consumed.

7. Food composition for its use as claimed in any one of claims 3 to 6, **characterized in that** the food composition also contains at least one element selected from the group comprising fatty acids, vitamins, trace elements and mineral salts, and mixtures thereof.

8. Food composition for its use as claimed in any one of claims 3 to 7, **characterized in that** it contains at least one element chosen from the group comprising calcium, phosphorus, vitamin B1, vitamin B2, vitamin B12, vitamin B19, vitamin A and vitamin D, and mixtures thereof.

9. Food composition for its use as claimed in any one of claims 3 to 8, **characterized in that** the food composition is also flavored and/or colored.

10. Food composition for its use as claimed in any one of claims 3 to 8, **characterized in that** it improves and/or generally promotes the intestinal well-being of infants.
